# EUROPÄISCHE PATENTANMELDUNG

(11) **EP 3 533 421 A1**
(43) Veröffentlichungstag der Anmeldung: **04.09.2019**
(21) Anmeldenummer: 18159650.3
(22) Anmeldetag: 02.03.2018
(51) Int. Cl.: A61F 2/966

(54) **REIBKRAFTMINIMIERUNG DURCH UNTERBINDUNG VON PROTEINADHÄSION**

(71) Anmelder: Biotronik AG, 8180 Bülach (CH)
(72) Erfinder: Dietsche, Claudius, 79415 Bad Bellingen (DE); Westhoff, Felix, 8057 Zürich (CH); Rothfuchs, Nuria, 8032 Zürich (CH)
(74) Vertreter: Randoll, Sören

(57) **Zusammenfassung**

Die Erfindung betrifft eine Kathetereinrichtung zum Implantieren eines medizinischen Implantats, insbesondere eines selbstexpandierenden Stents, in einem Lumen eines Patienten.

## Beschreibung

Die Erfindung betrifft eine Kathetereinrichtung zum Implantieren eines medizinischen Implantats, insbesondere eines selbstexpandierenden Stents, in einem Lumen eines Patienten.

Im Hinblick auf das Implantieren von Implantaten, insbesondere von selbstexpandierenden Stents, mittels einer Kathetereinrichtung ist es wünschenswert eine möglichst geringe Stentfreisetzkraft sowie Reibungskraft zwischen dem Stent und einem Außenschaft der Kathetereinrichtung zu erhalten.

Bekannte Lösungen sehen zum Verringern dieser Stentfreisetzkraft z. B. die Erhöhung einer Spaltbreite zwischen den reibenden Komponenten (z. B. Außenschaft zu Innenschaft) vor.

Weiterhin werden Oberflächenbehandlungen vorgeschlagen, die die besagte Reibung, vor allem aber die Reibung zwischen der Außenseite des Implantats und der Innenseite des Außenschafts vermindern.

Ferner existieren alternative Lösungsvorschläge, die eine Erhöhung der maximalen Kraftgrenze durch stärkere Materialien vorsehen. Dies kann zum Beispiel dadurch geschehen, dass die Komponenten stärker ausgelegt werden, um die Reibung zu vertragen. Nachteilig ist dabei allerdings, dass die stärkeren Komponenten mehr Platz beanspruchen und die Biegesteifigkeit (Trackability) leidet.

Hinsichtlich der Verringerung der Reibungskraft durch Erhöhung der besagten Spaltbreite ergibt sich allerdings die Problematik, dass bei Katheter-Systemen (z. B. 4 French) grundsätzlich Limitierungen des Innendurchmessers (Guidewirekompatibilität) und des maximalen Außendurchmessers (Introducer- /Führungskatheterkompatibilität) vorhanden sind, so dass sich die Erhöhung der Spaltbreite schwierig gestaltet. Insbesondere führt eine Verringerung von Materialwandstärken zur Erzielung einer höheren Spaltbreite zu einer reduzierter Stabilität und Sicherheit.

Hinsichtlich der Vornahme von Oberflächenbehandlungen zur Reduktion der unerwünschten Reibung bestehen die Nachteile, dass diese regelmäßig verhältnismäßig hohe Kosten bedingen, da es sich um vergleichsweise zeitintensive Prozesse handelt. Hierbei erweist es sich insbesondere als schwierig eine hohe Homogenität der Behandlung sicherzustellen bzw. zu prüfen.

Schließlich erweist sich die Erhöhung der maximalen Kraftgrenze durch stärkere Materialien insoweit als Nachteil, da hier die Freisetzkraft unverändert hoch ist und des Weiteren der Komfort der Einrichtung entsprechend beeinträchtigt ist (höhere notwendige Kraftaufbringung vom Arzt bzw. Verwender der Einrichtung). Die Erhöhung der maximalen Kraftgrenze ist ohnehin limitiert, da das schwächste Element im Katheter die maximale Kraft beschränkt.

Der Erfindung liegt daher die Aufgabe zugrunde, eine Kathetereinrichtung bereitzustellen, bei der die vorstehend beschriebene Reibung, insbesondere die Stentfreisetzungskraft, verringert ist.

Diese Aufgabe wird durch eine Kathetereinrichtung mit den Merkmalen des Anspruchs 1 gelöst. Vorteilhafte Ausführungsformen der Erfindung sind in den entsprechenden Unteransprüchen vorgesehen und werden nachfolgend beschrieben.

Es wird eine Kathetereinrichtung mit einem Außenschaft und einem medizinischen Implantat vorgeschlagen, wobei ein distaler Endabschnitt des Außenschafts dazu ausgebildet ist, das medizinische Implantat zum Transportieren des Implantats an einem Implantationsort aufzunehmen, wobei das Implantat in einem von dem Endabschnitt umgebenden Lumen des Außenschaftes anordenbar ist, und wobei der distale Endabschnitt des Außenschaftes und das Implantat zum Freisetzen des Implantats am Implantationsort relativ zueinander bewegbar sind. Dabei kann beispielsweise das Ende des Außenschaftes als Kapsel oder als eigentliches Schlauchende ausgebildet sein.

Erfindungsgemäß ist nun vorgesehen, dass zur Reduktion einer Reibung zwischen dem Endabschnitt des Außenschaftes und dem Implantat eine proteinpassivierende Verbindung in das Lumen am distalen Endabschnitt eingebracht ist. Protein passivierend soll dabei bedeuten, dass die Anlagerung von Proteinen an die mit der proteinpassivierenden Verbindung umgebenen Oberflächen verhindert wird. Ohne an diese Theorie gebunden zu sein wird angenommen, dass die proteinpassivierende Verbindung an reibungsfördernde Proteine anlagern bevor die Proteine an Oberflächen des Implantats anbinden können und dadurch eine signifikante Erhöhung der Reibung zwischen dem Endabschnitt des Außenschaftes und dem Implantat verhindern.

Eine hydrophile bzw. gut wasserlösliche Verbindung im Sinne der Erfindung ist insbesondere durch eine Löslichkeit von mindestens 10 g/L [Gramm/Liter] in Wasser bei 15°C bis 25°C gekennzeichnet, insbesondere mindestens 30 g/L, insbesondere mindestens 100g/L.

Die Erfindung sieht also mit anderen Worten eine Reduzierung der Reibungskraft In Vivo durch Anwendung von einer proteinpassivierenden Verbindung vor, insbesondere durch Aufbringen von Polyethylenglykol (PEG) in den distalen Schaftbereich (siehe unten).

Der Erfindung liegt die überraschende Entdeckung zugrunde, dass bei einem Blutkontakt die Oberflächen der Kathetereinrichtung, insbesondere der Innenseite des Außenschaftes und der Außenseite des Implantats bzw. Stents mit Plasmaproteinen, also mit Proteinen aus dem Blutplasma, sehr schnell bedeckt werden, wobei die Anhaftung von Proteinen an den Stent- und Schaftoberflächen zu einer starken Zunahme der Reibung bei Relativbewegungen führt. Diese geschwindigkeitsabhängige Erhöhung der Reibung durch Proteinanhaftung zeigt sich insbesondere bei geringen Abständen (immer gegeben bei selbstexpandierenden Implantaten) zwischen zwei sich relative zueinander bewegenden Oberflächen. Eine solche starke Einflussnahme der Proteinanlagerung auf die Schiebereibung bei der eigentlich kurzen Exposition des Katheters während der Implantierungsprozedur war durchaus überraschend und konnte in dem Ausmaß nicht vorhergesehen werden.

Die vorstehend beschriebenen, bekannten Lösungen unterbinden die Proteinanhaftung und den entsprechenden Reibungsanstieg hingegen nicht und adressieren auch die Problemstellung einer sich signifikant auswirkenden Proteinanlagerung nicht.

Durch die erfindungsgemäße Einbringung einer proteinpassivierenden Verbindung, (z. B. PEG) in den distalen Außenschaft wird die Adsorption von Blutproteinen an Oberflächen verringert oder verhindert. Dies führt zu einer reduzierten Reibung zwischen Implantat und Außenschaft, was wiederum die Kraft während der Stentfreisetzung verringert.

Gemäß einer Ausführungsform der Erfindung ist vorgesehen, dass die proteinpassivierende Verbindung biokompatibel ist.

Weiterhin ist gemäß einer Ausführungsform der Erfindung vorgesehen, dass das medizinische Implantat als ein Stent ausgebildet ist, der insbesondere aus einer Vielzahl mit einander (insbesondere einstückig) verbundender Streben gebildet ist, die eine Vielzahl an Zellen ausbilden. Der Stent kann selbstexpandierbar ausgebildet sein, so dass er sich beim Freisetzen selbsttätig von einem komprimierten Durchmesser (Stent ist im Außenschaft aufgenommen) auf einen Enddurchmesser entfaltet.

Weiterhin ist gemäß einer Ausführungsform der Erfindung vorgesehen, dass die proteinpassivierende Verbindung auf eine Innenseite des Endabschnitts des Außenschaftes aufgebracht ist. Diese Innenseite ist dem Lumen des Außenschaftes zugewandt und umläuft das im Lumen angeordnete Implantat entsprechend. Diese Innenseite ist daher auch einer Außenseite des Implantats zugewandt.

Alternativ oder ergänzend ist weiterhin gemäß einer Ausführungsform der Erfindung vorgesehen, dass die proteinpassivierende Verbindung auf die besagte Außenseite des Implantats aufgebracht ist.

Weiterhin können die besagte Innenseite und/oder die besagte Außenseite mit der proteinpassivierenden Verbindung (insbesondere dauerhaft) beschichtet sein.

Weiterhin ist gemäß einer Ausführungsform der Erfindung vorgesehen, dass die proteinpassivierende Verbindung ein hydrophiles Polymer aufweist oder durch ein hydrophiles Polymer gebildet ist. Gemäß einer weiteren Ausführungsform weist das hydrophile Polymer eine Vielzahl von Hydroxidgruppen auf. In einer bevorzugten Ausführungsform ist die proteinpassivierende Verbindung ausgesucht aus der Gruppe umfassend oder bestehend aus Polyethylenglykol (PEG), Polypropylenglykol, sowie weitere Alditole mit einer linearen C5 bis C25 Kohlenstoffkette.

Weiterhin ist gemäß einer Ausführungsform der Erfindung vorgesehen, dass die proteinpassivierende Verbindung Polyethylenglykol (PEG) aufweist oder durch PEG gebildet wird. Insbesondere weist die Verbindung kurzkettiges, niedermolekulares PEG auf, vorzugsweise mit einem Molekulargewicht zwischen 100 bis 800 g/mol und weiter bevorzugt mit einem Molekulargewicht von 200 bis 500 g/mol.
Weiterhin ist gemäß einer Ausführungsform der Erfindung vorgesehen, dass es sich bei dem PEG um PEG mit einem Molekulargewicht von 350 bis 450 g/mol handelt.

Durch das teilweise amphipatische Verhalten von Proteinen ist eine Bindung alternativ auch durch hydrophobe Verbindungen möglich. In einer weiteren Ausführungsform ist die proteinpassivierende Verbindung ausgesucht aus der Gruppe umfassend oder bestehend aus Silikonöl, Paraffinöl und niedermolekulare flüssige Silikone, vorzugsweise mit einem Molekulargewicht von unter 150000 g/mol.

Weiterhin ist gemäß einer Ausführungsform der Erfindung vorgesehen, dass die Kathetereinrichtung einen Innenschaft aufweist, der im Lumen des Außenschaftes angeordnet ist, wobei das Implantat auf einem distalen Endabschnitt des Innenschaftes anordenbar ist, und wobei der Innenschaft und der Außenschaft zum Freisetzen des Implantats relativ zueinander verschiebbar sind.

Weiterhin kann auf dem Innenschaft ein Stopper (bzw. Stopperschaft) angeordnet sein, der am Innenschaft umläuft und eine Anlagefläche für ein proximales Ende des Stents bildet.

Weiterhin kann am distalen Ende des Innenschaftes eine Katheterspitze vorgesehen sein, die den Außenschaft am distalen Ende verschließt, wenn das im Außenschaft aufgenommene Implantat mittels des Außenschaftes an den Implantationsort gebracht wird.

Beim relativen Verschieben des Außenschaftes und des Innenschaftes wird das Implantat aus dem Außenschaft heraus bewegt und entfaltet sich dabei insbesondere selbsttätig. Der Stopper kann hierbei ein Widerlager am proximalen Ende des Stents bilden, so dass der Stent bezüglich des Innenschaftes nicht verschoben wird, wenn der Außenschaft gegenüber dem Innenschaft zurückgezogen wird bzw. der Innenschaft mit dem Stent aus dem Außenschaft herausgeschoben wird.

Im Sinne der vorliegenden Erfindung bezieht sich der Begriff distal auf eine Komponente der Kathetereinrichtung, die entlang der Kathetereinrichtung weiter von dem Arzt/Verwender bzw. Handgriff der Kathetereinrichtung entfernt ist als eine proximale Komponente.

Weiterhin kann der Innenschaft (sowie die Katheterspitze) jeweils ein Lumen aufweisen, durch das sich ein Führungsdraht erstrecken kann. Mittels des Führungsdrahtes kann der Außenschaft mit dem Implantat beim Implantieren zum Implantationsort geführt werden.

Gemäß einem weiteren Aspekt der vorliegenden Erfindung wird ein Verfahren zur Herstellung einer erfindungsgemäßen Kathetereinrichtung offenbart, wobei in ein Lumen eines distalen Endabschnitts eines Außenschaftes der Kathetereinrichtung eine proteinpassivierende Verbindung zur Reduktion einer Reibung zwischen einer Innenseite des Endabschnitts des Außenschaftes und einem im Lumen des Endabschnitts des Außenschaftes angeordneten medizinischen Implantat eingebracht wird. Bei dem Implantat handelt es sich bevorzugt um einen Stent.

Weiterhin kann die proteinpassivierende Verbindung auch in einen Spalt zwischen dem Außenschaft und dem Innenschaft eingebracht werden.

Die bei dem Verfahren verwendete proteinpassivierende Verbindung kann im Einzelnen die oben bereits dargestellten Merkmale und Eigenschaften aufweisen und kann wie oben beschrieben in das Lumen bzw. auf die Innenseite des Endabschnitts des Außenschaftes bzw. auf die Außenseite des Implantats/Stents aufgebracht werden. Insbesondere handelt es sich der proteinpassivierenden Verbindung um PEG (siehe oben).

Demgemäß betrifft ein weiterer Aspekt der vorliegenden Erfindung die Verwendung einer hydrophilen oder hydrophoben chemischen Verbindung, wie hierin beschrieben und insbesondere von PEG, bevorzugt mit einem Molekulargewicht im Bereich von 200 bis 500 g/mol, als Mittel zur Verhinderung von Proteinanlagerung in Kathetersystemen, insbesondere als Mittel zur Verhinderung von Proteinanlagerung auf einem Implantat und einem Außenschaft eines implantierbaren Katheters.

Durch die erfindungsgemäße Verringerung der Reibungskraft und somit der Maximalkraft während des Freisetzens des Implantats kann die Kathetereinrichtung mit einem geringeren Durchmesser des Außenschaftes konstruiert werden, um dieselbe Stabilität bzw. Sicherheit zu gewährleisten. Des Weiteren können die Spaltmaße zwischen zwei Schläuchen bzw. zwischen Außen- und Innenschaft noch geringer gewählt werden, was wiederum einer Verkleinerung des gesamten Systems oder eine Erhöhung der Stabilität entspricht.

Die Proteinanhaftung, insbesondere von Proteinen aus dem Blutplasma, führt zu einer Streuung der Reibung bei der Freisetzung von Implantaten. Eine Streuung erfordert eine konstruktive Lösung, welche auf ein vielfaches des Kraftdurchschnittes ausgelegt sein muss, um die gewünschte Sicherheit zu gewährleisten. Bei Verhinderung der Proteinanhaftung wird die Streuung reduziert und dadurch der Risikofaktor für einen (statistischen) Ausreißer drastisch verringert.

Eine geringe Maximalkraft während der Freisetzung erhöht den Komfort einer Kathetereinrichtung. Dies ist möglich, da die Maximalkraft sinkt und somit Bedienkräfte sinken.

Teure Prozessschritte (z. B. Oberflächenbehandlungen, Prüfungen) sind nicht mehr nötig und reduzieren die Kosten für die Produktion. Da geringere Kräfte wirken, kann die Qualität (z. B. Toleranzen) von den Materialien angepasst werden, was wiederum Kosten einspart.

Im Folgenden sollen weitere Merkmale sowie Ausführungsformen/Beispiele der Erfindung anhand der Figuren erläutert werden. Es zeigen:
- Fig. 1: eine schematische Schnittdarstellung einer erfindungsgemäßen Kathetereinrichtung beim Transportieren eines im Außenschaft angeordneten Implantats zum Implantationsort;
- Fig. 2: eine schematische Schnittdarstellung der in der Figur 1 gezeigten Kathetereinrichtung beim Freisetzen des Implantats;
- Fig. 3: eine Messanordnung zum Messen der Reibungskraft zwischen dem Implantat und dem Außenschaft;
- Fig. 4: Messergebnisse für die gemäß Figur 3 gemessene Reibungskraft;
- Fig. 5: eine Messanordnung zum Messen der Reibung im Gesamtsystem; und
- Fig. 6: Messergebnisse der nach Figur 5 gemessenen Reibung.

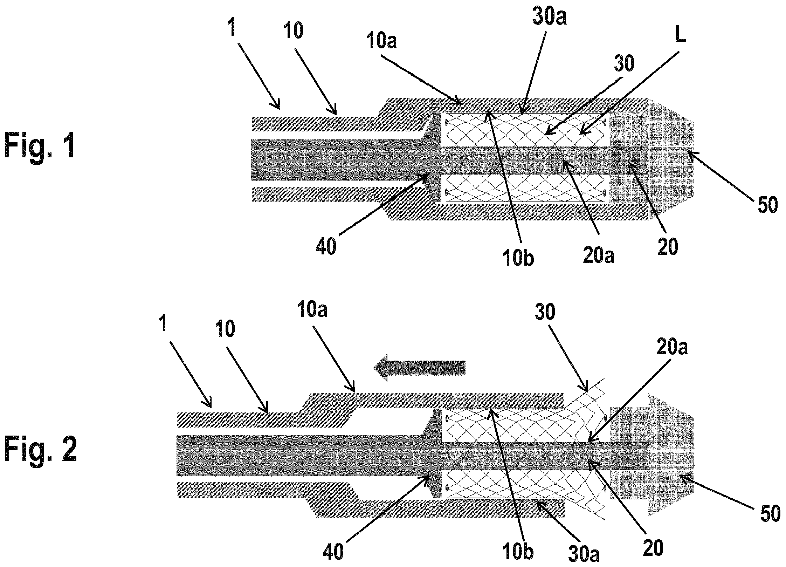

Die Figur 1 zeigt im Zusammenhang mit Figur 2 eine erfindungsgemäße Kathetereinrichtung 1 mit einem Außenschaft 10 und einem medizinischen Implantat 30, hier in Form eines selbstexpandierenden Stents 30, wobei ein distaler Endabschnitt 10a des Außenschafts 10 dazu ausgebildet ist, das Implantat 30 zum Transportieren des Implantats 30 an einem Implantationsort aufzunehmen, wobei das Implantat 30 in einem von dem Endabschnitt 10a umgebenden Lumen L des Außenschaftes 10 anordenbar ist. Beim Transport zum Zielort kann das Lumen L des Außenschaftes 10 distal mit einer Katheterspitze 50 verschlossen sein. Zum Freisetzen des Stents 30 wird der distale Endabschnitt 10a des Außenschaftes 10 und das Implantat 30 am Implantationsort relativ zueinander bewegt (vgl. Fig. 2). Hierbei ist zur Reduktion einer Reibung zwischen dem Endabschnitt 10a des Außenschaftes 10 und dem Implantat 30 eine proteinpassivierende Verbindung wie hierin beschrieben in das Lumen am distalen Endabschnitt 10a des Außenschaftes 10 eingebracht.

Zum Freisetzen des Stents 30 weist die Kathetereinrichtung 1 bevorzugt einen Innenschaft 20 auf, der im Lumen L des Außenschaftes 10 angeordnet ist, wobei das Implantat 30 auf einem distalen Endabschnitt 20a des Innenschaftes 20 angeordnet. Werden nun der Innenschaft 20 und der Außenschaft 10 gegeneinander verschoben, wie es in der Figur 2 gezeigt ist, wird der Stent 30 aus dem distalen Endabschnitt 10a des Außenschaftes heraus bewegt, wodurch der Stent 30 freigesetzt wird und sich entsprechend entfaltet (hier z. B. selbstexpandierend). Auf dem Innenschaft 20 ist bevorzugt ein sogenannter Stopper 40 angeordnet, der am Innenschaft 20 umläuft und eine Anlagefläche für ein proximales Ende des Stents 30 bildet. Der Stopper hält den Stent auf dem Innenschaft in Position während der Außenschaft 10 zurückgezogen bzw. der Innenschaft 20 vorgeschoben wird.

Insbesondere beim Freisetzen des Stents 30 findet eine gegenläufige Bewegung zwischen der Innenseite 10b des Endabschnitts 10a des Außenschaftes 10 und einer Außenseite 30a des Stents 30 statt, die eine entsprechende Reibung erzeugt.

Gemäß einem Beispiel der Erfindung wird diese durch Proteinanlagerung bedingte Reibung (siehe auch oben) durch Einbringen einer proteinpassivierenden Verbindung in Form von PEG in das Lumen L im Bereich des Endabschnitts 10a des Außenschaftes 10 verringert.

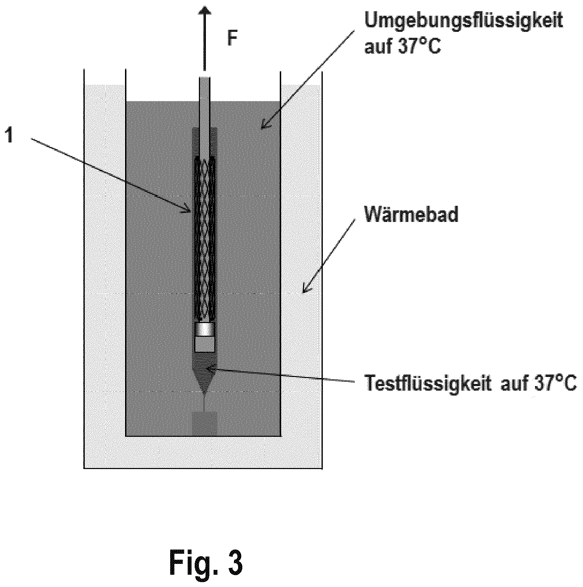

Dies wird anhand der in den Figuren 3 bis 6 dargestellten Messungen ersichtlich.

Hierzu wurde gemäß Figur 3 die Kathetereinrichtung 1 in einen Behälter mit einer Umgebungsflüssigkeit gegeben, wobei das Lumen die jeweilige Testflüssigkeit, insbesondere PEG, enthielt. Sodann wurde die Kraft zum Herausziehen des Stents 30 aus dem Außenschaft 10 gemäß Figur 3 gemessen.

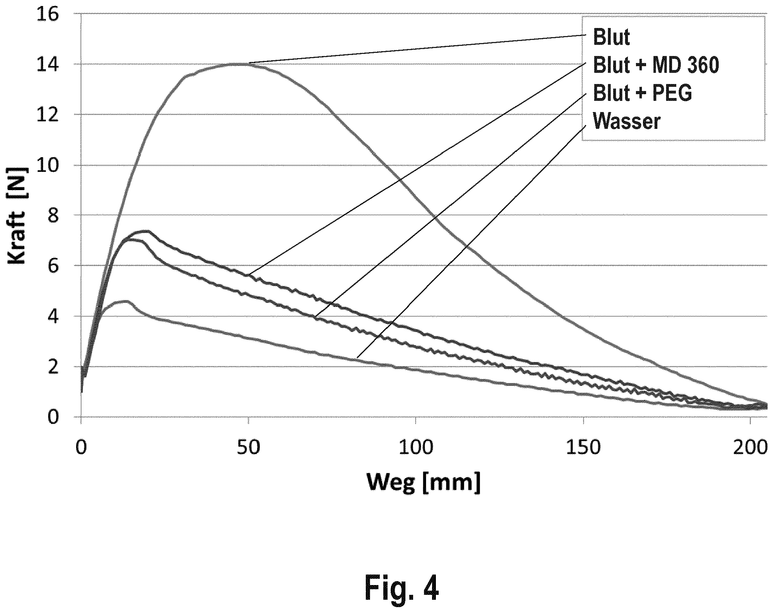

Figur 4 zeigt diesbezüglich eine deutliche Reduktion der gemessenen Kraft für eine Blut und PEG (hier mit einer Konzentration von 1-3 Volumen-%) aufweisende Testflüssigkeit gegenüber der aus Blut bestehenden Testflüssigkeit. Es wird klar ersichtlich, dass durch die Einbringung einer proteinpassivierenden Verbindung, hier PEG oder das Silikonöl MD 360 die Erhöhung der Reibung durch Proteinanlagerung wirksam verringert und annähernd verhindert wird.

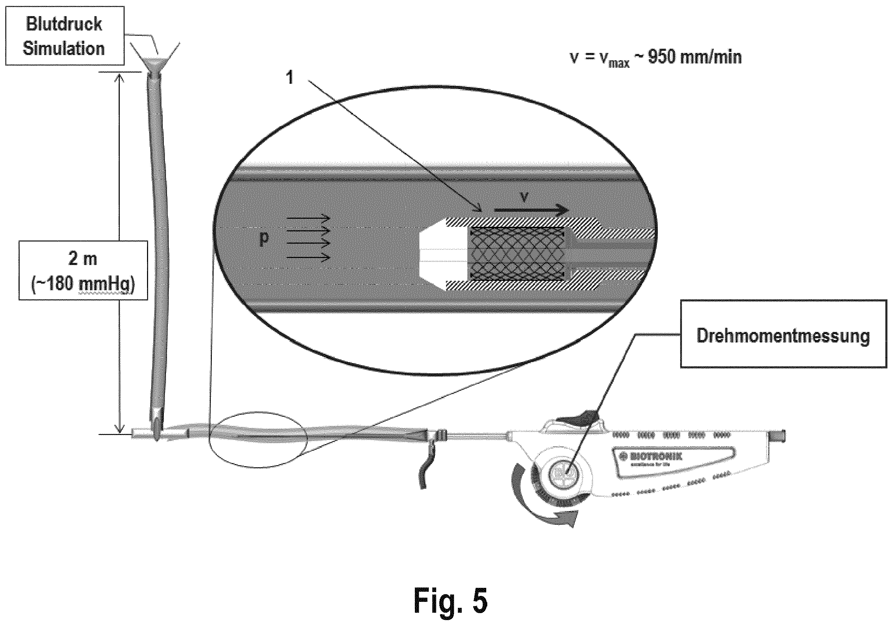

Weiterhin wurde die Gesamtreibung im System anhand der in der Figur 5 dargestellten Messanordnung gemessen. Hierbei wurde der Stent 30 in einem simulierten Blutgefäß freigesetzt, wobei ein Drehmoment eines Betätigungselementes der Kathetereinrichtung 1 gemessen wurde. Das Betätigungselement ist hierbei rotierbar, wobei die Rotation des Betätigungselementes in eine lineare Bewegung des Stents 30 relativ zum Außenschaft10 umgesetzt wird.

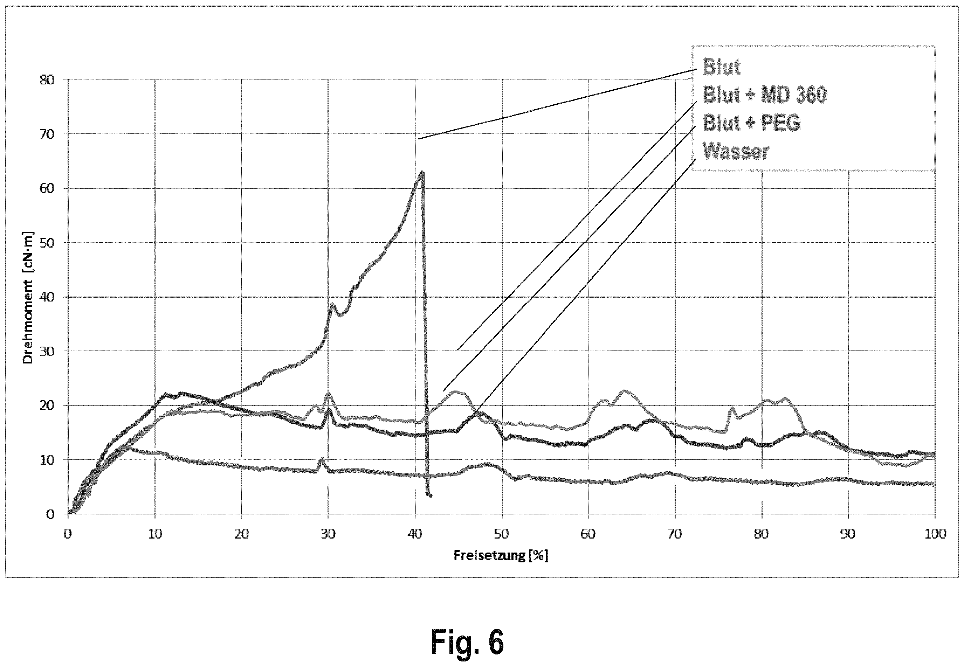

Wie anhand der Figur 6 ersichtlich ist, ist das Drehmoment bei der Freisetzgeschwindigkeit (Bewegungsgeschwindigkeit vom Außenschaft relativ zum Implantat) v₂ reduziert sofern zuvor PEG in das Lumen L am Endabschnitt 10a eingebracht wurde.

Des Weiteren wurden Untersuchungen durchgeführt, die den Unterschied zwischen der Freisetzung in einer Mischung aus Wasser und PEG, in Blut mit PEG und in Blut alleine zum Gegenstand hatten. In diesen Untersuchung hat sich gezeigt, dass die beide Freisetzungen mit PEG unter dem gleichen Kraftaufwand durchgeführt werden können und eine maximale Kraftaufwendung von ca. 24 N·cm benötigen, wobei die Kraftaufwendung nach der ersten Umdrehung am Betätigungselementes der Kathetereinrichtung 1 deutlich bis zur endgültigen Freisetzung abnimmt. Im Gegensatz dazu zeigte sich bei der Freisetzung in Blut ohne PEG, dass die Kraftaufwendung zur Freisetzung stetig zunimmt und nach knapp zwei vollständigen Umdrehungen bei ca. 65 N·cm liegt, bevor das System nicht weiter freigesetzt werden konnte.

## Patentansprüche

1. Kathetereinrichtung (1) mit einem Außenschaft (10) und einem medizinischen Implantat (30), wobei ein distaler Endabschnitt (10a) des Außenschafts (10) dazu ausgebildet ist, das Implantat (30) zum Transportieren des Implantats (30) an einem Implantationsort aufzunehmen, wobei das Implantat (30) in einem von dem Endabschnitt (10a) umgebenden Lumen (L) des Außenschaftes (10) anordenbar ist, und wobei der distale Endabschnitt (10a) des Außenschaftes (10) und das Implantat (30) zum Freisetzen des Implantats (30) am Implantationsort relativ zueinander bewegbar sind,
**dadurch gekennzeichnet,**
**dass** zur Reduktion einer Reibung zwischen dem Endabschnitt (10a) des Außenschaftes (10) und dem Implantat (30) eine proteinpassivierende Verbindung in das Lumen (L) am distalen Endabschnitt (10a) des Außenschaftes (10) eingebracht ist.

2. Kathetereinrichtung nach Anspruch 1, **dadurch gekennzeichnet, dass** die proteinpassivierende Verbindung auf eine Innenseite (10b) des Endabschnitts (10a) des Außenschaftes (10) aufgebracht ist.

3. Kathetereinrichtung nach Anspruch 1 oder 2, **dadurch gekennzeichnet, dass** die proteinpassivierende Verbindung auf eine Außenseite (30a) des Implantats (30) aufgebracht ist.

4. Kathetereinrichtung nach Anspruch 2 oder 3, **dadurch gekennzeichnet, dass** die Innenseite (10b) und/oder die Außenseite (30a) mit der proteinpassivierenden Verbindung beschichtet ist.

5. Kathetereinrichtung nach einem der vorhergehenden Ansprüche, **dadurch gekennzeichnet, dass** die proteinpassivierende Verbindung ein hydrophiles oder hydrophobes Polymer aufweist oder durch ein hydrophiles oder hydrophobes Polymer gebildet ist.

6. Kathetereinrichtung nach einem der vorhergehenden Ansprüche, **dadurch gekennzeichnet, dass** die Verbindung PEG aufweist oder durch PEG gebildet wird.

7. Kathetereinrichtung nach Anspruch 6, **dadurch gekennzeichnet, dass** es sich bei dem PEG um niedermolekulares PEG handelt.

8. Kathetereinrichtung nach einem der vorhergehenden Ansprüche, **dadurch gekennzeichnet, dass** das Implantat (30) ein Stent ist.

9. Kathetereinrichtung nach einem der vorhergehenden Ansprüche, **dadurch gekennzeichnet, dass** die Kathetereinrichtung (1) einen Innenschaft (20) aufweist, der im Lumen (L) des Außenschaftes (10) angeordnet ist, wobei das Implantat (30) auf einem distalen Endabschnitt (20a) des Innenschaftes (20) anordenbar ist, und wobei der Innenschaft (20) und der Außenschaft (10) zum Freisetzen des Implantats (30) relativ zueinander verschiebbar sind.

10. Kathetereinrichtung nach Anspruch 9, **dadurch gekennzeichnet, dass** die proteinpassivierende Verbindung in einen Spalt zwischen dem Innenschaft (20) und dem Außenschaft (10) eingebracht ist.

11. Verfahren zur Herstellung einer Kathetereinrichtung, wobei in ein Lumen (L) eines distalen Endabschnitts (10a) eines Außenschaftes (10) der Kathetereinrichtung (1) eine proteinpassivierende Verbindung zur Reduktion einer Reibung zwischen einer Innenseite (10b) des Endabschnitts (10a) des Außenschaftes (10) und einem im Lumen (L) des Endabschnitts (10a) des Außenschaftes (10) angeordneten medizinischen Implantat (30) eingebracht wird.
